(19) Europäisches Patentamt
European Patent Office
Office européen des brevets

(11) Numéro de publication: **0 351 282 B1**

# (12) FASCICULE DE BREVET EUROPEEN

(45) Date de publication de fascicule du brevet: **27.10.93**

(51) Int. Cl.⁵: **C07D 401/06**, C07D 491/10, A61K 31/40

(21) Numéro de dépôt: **89401898.5**

(22) Date de dépôt: **03.07.89**

(54) **Dérivés de (hydroxy-1 pipéridinyl-2 alkyl)indolones-2, quinoléinones-2, benzo[b]azépinones-2, benzimidazolones-2 et quinazolinones-2, leur préparation et leur application en thérapeutique.**

(30) Priorité: **12.07.88 FR 8809449**
**13.12.88 FR 8816373**

(43) Date de publication de la demande:
**17.01.90 Bulletin 90/03**

(45) Mention de la délivrance du brevet:
**27.10.93 Bulletin 93/43**

(84) Etats contractants désignés:
**AT BE CH DE ES FR GB GR IT LI LU NL SE**

(56) Documents cités:
**EP-A- 0 099 766**
**EP-A- 0 202 164**
**GB-A- 2 071 094**

**CHEMICAL ABSTRACTS, vol. 87, 1977, page 453, abstract no. 53098r, Columbus, Ohio, US**

(73) Titulaire: **SYNTHELABO**
**22, avenue Galilée**
**F-92350 Le Plessis-Robinson(FR)**

(72) Inventeur: **Frost, Jonathan**
**23, Route de Montjean**
**F-91320 Wissous(FR)**
Inventeur: **Lardenois, Patrick**
**18, Rue Varengue**
**F-92340 Bourg la Reine(FR)**
Inventeur: **Bertin, Jean**
**55, rue d'Estienne d'Orves**
**F-92140 Clamart(FR)**
Inventeur: **Saarmets, Alfred**
**35bis, rue du Moulin à Vent**
**F-94370 Sucy en Brie(FR)**
Inventeur: **Rousselle, Corinne**
**Le Manoir Bosc Bernard Crescy**
**F-27310 bourg Achard(FR)**

(74) Mandataire: **Ludwig, Jacques et al**
**SYNTHELABO,**
**Service Brevets,**
**B.P. 72**
**F-92352 Le Plessis-Robinson Cédex (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 351 282 B1

Rank Xerox (UK) Business Services
(3.10/3.6/3.3.1)

## Description

La présente invention a pour objet des dérivés de (hydroxy-1 pipéridinyl-2 alkyl) indolones-2, quinoléinones-2, benzo[*b*]-azépinones-2, benzimidazolones-2 et quinazolinones-2, leur préparation et leur application en thérapeutique.

Les composés de l'invention répondent à la formule générale (I) du schéma ci-après, formule dans laquelle
Z représente un groupe de formule $-CH_2-$, $-C(CH_3)_2-$, $-CH=CH-$, $-(CH_2)_2-$, $-(CH_2)_3-$, -NH- ou $-N(CH_3)CH_2-$ (dont l'atome d'azote est lié au groupe carbonyle),
R1 représente un atome d'hydrogène ou un groupe alkyle en $C_1$-$C_4$,
R2 représente un atome d'hydrogène ou un groupe méthyle, et
R3 représente soit un groupe phénoxy éventuellement substitué par un atome d'halogène ou un groupe méthyle, soit un groupe naphtyloxy, soit un groupe phénylméthyle substitué par un atome d'halogène ou un groupe méthyle, soit un groupe phénylméthyle non substitué lorsque Z ne représente pas un groupe de formule $-CH=CH-$ ou $-(CH_2)_2-$, soit un groupe bis(fluoro-4 phényl)méthyle, soit un groupe phénylméthoxy éventuellement substitué par un atome d'halogène ou un groupe méthyle, soit un groupe (naphtyl-2)-méthoxy, soit un groupe phénoxyméthyle éventuellement substitué par un atome d'halogène ou un groupe méthyle, soit un groupe pyridinyloxy, et
R4 représente un atome d'hydrogène, ou bien encore
R3 et R4 forment, ensemble et avec le cycle de pipéridine, un groupe spiro(dihydro-2,3 benzofuranne-2:4'-pipéridinyle-1').

Des composés analogues à ceux de la présente invention sont décrits dans la demande de brevet GB-2071094, notamment comme antihistaminiques et médicaments du système nerveux central. D'autres composés analogues sont décrits dans C.A., 86, 189739n, C.A., 87, 53098r, dans le brevet US-4711899, et dans la demande de brevet EP-0099766.

Les composés de l'invention peuvent se présenter sous forme de bases libres ou de sels d'addition à des acides.
Lorsque R2 désigne l'hydrogène, les molécules de formule (I) comportent un seul atome de carbone asymétrique. Ils peuvent donc se présenter sous la forme d'énantiomères purs ou de leurs mélanges.

Schéma

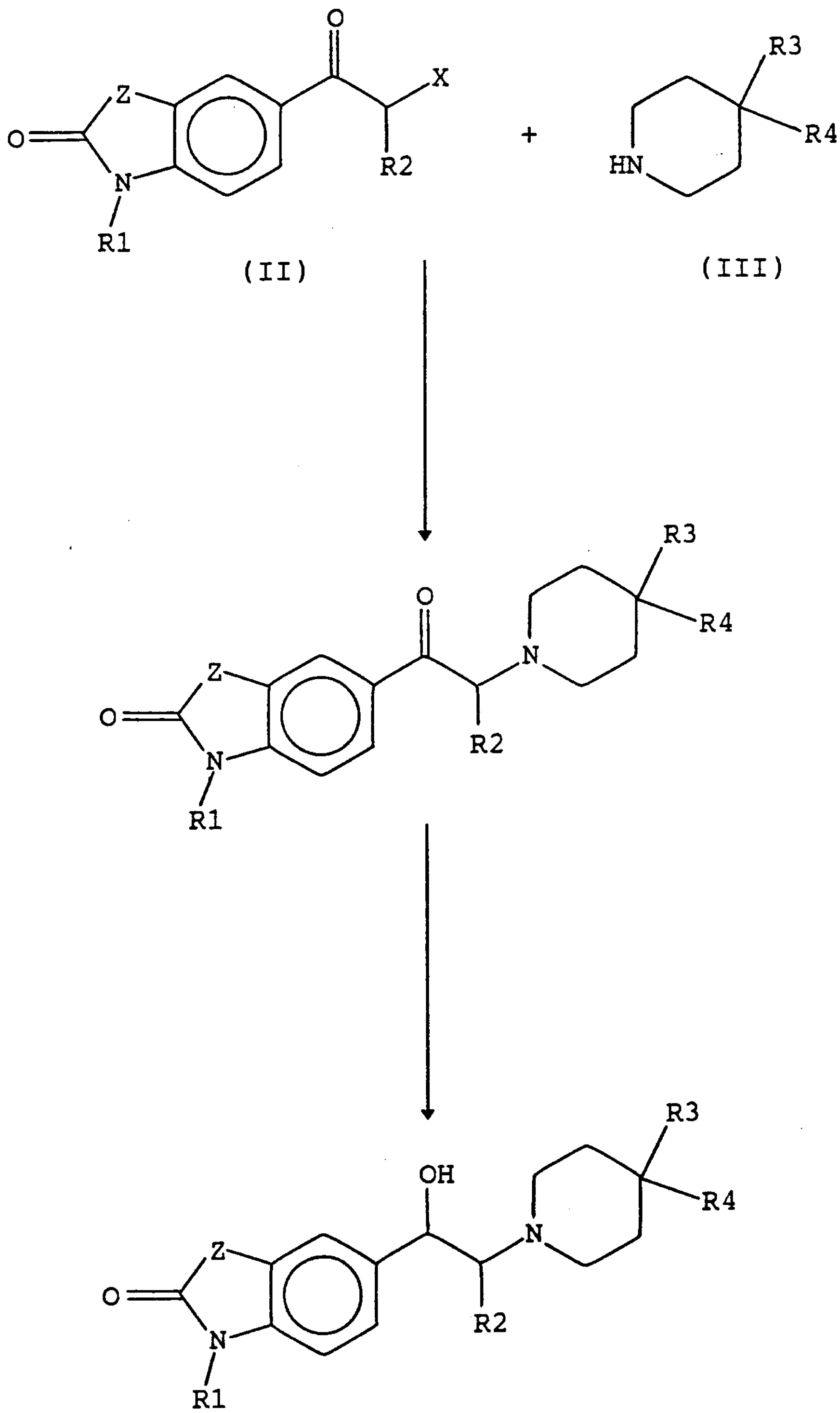

(II)

(III)

(IV)

(I)

Lorsque R2 désigne un groupe méthyle, les molécules de formule (I) comportent deux atomes de carbone asymétriques vicinaux. Il y a donc deux formes diastéréoisomères, érythro et thréo, dont chacune comprend deux énantiomères.

L'invention comprend chacune de ces formes pures, ainsi que leurs mélanges.

Conformément à l'invention on peut préparer les composés de formule (I) par un procédé illustré par le schéma qui précède.

On fait d'abord réagir une cétone halogénée de formule générale (II) (dans laquelle Z, R1 et R2 sont tels que définis ci-dessus et X représente un atome d'halogène tel que le chlore ou le brome) avec une pipéridine de formule générale (III) (dans laquelle R3 et R4 sont tels que définis ci-dessus), puis on réduit la cétone de formule générale (IV) ainsi obtenue.

Les deux étapes du procédé sont des réactions de types bien connus de l'homme de l'art.

La première étape, réaction entre un dérivé halogéné et une amine secondaire, peut se dérouler par exemple en présence d'une base minérale telle que le carbonate de sodium ou de potassium, ou en présence d'un excès de pipéridine de formule générale (III), dans un solvant tel qu'un alcool inférieur ou l'acétonitrile, et éventuellement en présence d'eau.

La deuxième étape, réduction d'une cétone en alcool, peut être effectuée par exemple au moyen de borohydrure de sodium ou de potassium, en milieu alcalin ou acide.

Les isomères optiques d'un composé de formule générale (I) peuvent être isolés à partir de leurs mélanges selon toutes méthodes connues.

Les cétones de formule générale (II) où Z représente $-CH_2-$ et R1 représente H peuvent être obtenues à partir de 3*H*-indolone-2 et de chlorure de chloroacétyle ou de chlorure de chloro-2 propanoyle, en présence de chlorure d'aluminium, comme décrit dans la demande de brevet européen N° 0168003.

Les cétones de formule générale (II) où Z représente $-C(CH_3)_2-$ et R1 représente H peuvent être obtenues, également, à partir de diméthyl-3,3 3*H*-indolone-2, décrite dans J. Med. Chem., 29, 1832-1840 (1986) par réaction avec le chlorure de chloroacétyle ou le chlorure de chloro-2 propanoyle, en présence de chlorure d'aluminium.

Les cétones de formule générale (II) où Z représente $-(CH_2)_2-$ou $-CH=CH-$ et R1 et R2 représentent chacun H sont décrites dans Chem. Pharm. Bull. 34(2), 682-693 (1986).

Celles où R2 représente $CH_3$ peuvent être obtenues, de manière analogue à la méthode connue, à partir de 1*H*-quinoléinone-2 et de chlorure de chloro-2 propanoyle.

Les cétones de formule générale (II) où Z représente un groupe de formule $-(CH_2)_2-$ peuvent être obtenues à partir de dihydro-3,4 1*H*-quinoléinone-2 et de chlorure de chloroacétyle ou de chlorure de chloro-2 propanoyle, en présence de chlorure d'aluminium, comme décrit dans C.A., 86, 189739n.

Les cétones de formule générale (II) où R1 représente un alkyle et Z représente un groupe de formule $-C(CH_3)_2-$,$-CH=CH-$ ou $-(CH_2)_2-$, peuvent, être obtenues par exemple à partir de diméthyl-3,3 3*H*-indolone-2, de 1*H*-quinoléinone-2 ou de dihydro-3,4 1*H*-quinoléinone-2 (décrites dans Zh. Org. Khim., 7(8), 1715-1721 (1971) et dans Rev. Latinoam. Quim., 9(4), 190-192 (1978)) par une alkylation classique, par exemple au moyen d'hydrure de sodium et d'un bromure d'alkyle.

Il va de soi, par ailleurs, que l'on peut préparer un composé de formule générale (I) où Z représente $-CH_2-CH_2-$ à partir du composé analogue où Z représente $-CH=CH-$, par une hydrogénation catalytique.

Les cétones de formule générale (II) où Z représente un groupe de formule $-(CH_2)_3-$ peuvent être obtenues, en deux étapes, d'abord à partir de dihydro-3,4 2*H*-naphtalénone-1 oxime, par réarrangement de Beckman, comme décrit dans J. Am. Chem. Soc., 74, 5153-5155(1952), et ensuite par action de chlorure de chloroacétyle ou de chlorure de chloro-2 propanoyle, dans les conditions indiquées ci-dessus.

Les cétones de formule générale (II) où Z représente un groupe de formule -NH- et R2 représente un atome d'hydrogène peuvent être obtenues à partir de benzimidazolone-2 et de chlorure de chloroacétyle, en présence de chlorure d'aluminium, comme décrit dans C.A., 101, 211043h ; celles où R2 représente un groupe méthyle peuvent être obtenues de manière analogue, par utilisation de chlorure de chloro-2 propanoyle au lieu de chlorure de chloroacétyle.

Les cétones de formule générale (II) où Z représente un groupe de formule $-N(CH_3)CH_2-$ peuvent être obtenues à partir de la méthyl-3 dihydro-3,4 1*H*-quinazolinone-2, décrite dans J. Het. Chem., 25, 789, (1988) et de chlorure de chloroacétyle ou chloro-2 propanoyle, en présence de chlorure d'aluminium, comme décrit dans Chem. Pharm. Bull., 36(6), 2253, (1988).

La plupart des pipéridines de formule générale (III) sont décrites dans la littérature. Celles où R3 représente un groupe phénoxy et R4 représente un atome d'hydrogène sont décrites dans J. Med. Chem., 17(9), 1000 (1974) ; celle où R3 représente un groupe naphtyloxy et R4 représente un atome d'hydrogène est décrite dans le brevet US-4443462 ; celles où R3 représente un groupe phénylméthyle substitué et R4 représente un atome d'hydrogène sont décrites dans le brevet EP-0106317 ; celles où R3 représente un groupe phénylméthoxy et R4 représente un atome d'hydrogène sont décrites dans le brevet EP-0077427 ; celle où R3 représente un groupe (naphtyl-2)méthoxy et R4 représente un atome d'hydrogène est décrite dans le brevet US-4529730 ; celles où R3 représente un groupe bis(fluoro-4 phényl)méthyle et R4 représente un atome d'hydrogène sont décrites dans le brevet BE-836394 ; celles où R3 représente un groupe pyridinyloxy et R4 représente un atome d'hydrogène peuvent être obtenues à partir de phénylméthyl-1 pipéridinol-4, d'abord par action de fluoro-2 pyridine en présence d'hydrure de sodium, puis

débenzylation catalytique de la phénylméthyl-1 (pyridinyl-2 oxy)-4 pipéridine intermédiaire ; celles où R3 représente un groupe phénoxyméthyle et R4 représente un atome d'hydrogène sont décrites dans C.A., 87, 84828h ; et enfin la spiro(dihydro-2,3 benzofuranne-2:4′-pipéridine) est décrite dans J. Het. Chem., 18(4), 811 (1981).

Les exemples qui vont suivre illustrent en détail la préparation de quelques composés selon l'invention. Les microanalyses et les spectres IR et RMN confirment les structures des produits obtenus.
Les numéros indiqués entre parenthèses dans les titres des exemples correspondent à ceux du tableau donné plus loin.

Exemple 1 (Composé n° 6).

(±)[[[(Fluoro-4 phényl)méthyl]-4 pipéridinyl-1]-2 hydroxy-1 éthyl]-5 3*H*-indolone-2.

a) Chloroacétyl-5 3*H*-indolone-2.

On agite pendant 15mn, à température ambiante, une suspension de 40 g (300 mmoles) de chlorure d'aluminium et 22,53 g, soit 15,9 ml (200 mmoles) de chlorure de chloroacétyle dans 60 ml de dichlorométhane.
Puis on ajoute, par petites portions, 13,32 g (100 mmoles) de 3*H*-indolone-2 et on chauffe le mélange au reflux pendant 40mn. On verse le mélange sur 800 ml de glace, on agite pendant 30mn, on sépare le solide par filtration, on le lave à l'eau, puis avec un peu d'éther, et on le sèche. On obtient 21,9 g de cristaux ocres qu'on utilise tels quels dans l'étape suivante.

b) (±)[[[(Fluoro-4 phényl)méthyl]-4 pipéridinyl-1]-2 hydroxy-1 éthyl]-5 3*H*-indolone-2.

On chauffe au reflux pendant 2h un mélange de 4,78 g (22,8 mmoles) de chloroacétyl-5 3*H*-indolone-2, 6,3 g (45,6 mmoles) de carbonate de potassium sec, 7,19 g (22,8 mmoles) de benzoate de (fluoro-4 phénylméthyl)-4 pipéridine et 60 ml d'éthanol.
On laisse refroidir le mélange, on ajoute 10 ml d'eau puis 5 g de borohydrure de potassium, on agite le mélange pendant 3h30 à température ambiante, on ajoute 200 ml d'eau et on le laisse reposer pendant 36h.
On ajoute de l'acétate d'éthyle, on agite, on sépare la phase organique, on extrait la phase aqueuse avec de l'acétate d'éthyle, on réunit les phases organiques, on lave à l'eau, sèche sur sulfate de sodium et évapore. On obtient 7,56 g de mousse rose qu'on purifie par chromatographie sur colonne de silice en éluant avec un mélange 96/4 de dichlorométhane/méthanol. On obtient ainsi 3,92 g de produit qu'on recristallise dans l'éthanol. On isole finalement 2,87 g de cristaux roses.
Point de fusion : 167-168 °C.

Exemple 2 (Composé n° 1).

(±)[Hydroxy-1 (phénoxy-4 pipéridinyl-1)-2 éthyl]-5 3*H*-indolone-2.

Sous atmosphère d'argon on chauffe au reflux pendant 1h15 un mélange de 4,19 g (20 mmoles) de chloroacétyl-5 3*H*-indolone-2, 4 g de carbonate de sodium sec, 4,27 g (20 mmoles) de chlorhydrate de phénoxy-4 pipéridine et 100 ml d'éthanol. On laisse refroidir le mélange, on ajoute 10 ml d'eau puis 8 g de borohydrure de potassium et on poursuit l'agitation pendant 2h30 à température ambiante. On ajoute encore 200 ml d'eau, on agite pendant 30 mn, on filtre le mélange, on lave le solide à l'eau et on le sèche. On obtient 4,67 g de cristaux ocres qu'on purifie par chromatographie sur colonne de silice en éluant avec un mélange 95/5 de dichlorométhane/méthanol.
On obtient ainsi 2,95 g de produit qu'on recristallise dans l'éthanol. On isole finalement 2 g de cristaux.
Point de fusion : 182-183 °C.

Exemple 3 (Composé n° 19).

(±)[Hydroxy-1 (phénylméthyl-4 pipéridinyl-1)-2 éthyl]-5 diméthyl-3,3 3*H*-indolone-2.

a) Chloroacétyl-5 diméthyl-3,3 3*H*-indolone-2.

A une suspension de 26,7 g (200 mmoles) de chlorure d'aluminium et 15 g, soit 10,58 ml (133 mmoles) de chlorure de chloroacétyle dans 60 ml de dichlorométhane, on ajoute lentement (en 1h) 10,8 g (66,4 mmoles) de diméthyl-3,3 3*H*-indolone-2, puis on chauffe le mélange au reflux pendant 1h30. On verse lentement la suspension brune obtenue sur 500 ml d'eau glacée, on agite le mélange pendant 30mn puis on le filtre, on lave le solide avec un peu d'éther et on le sèche. On obtient 16,3 g de cristaux qu'on utilise tels quels dans l'étape suivante.

b) (±)[Hydroxy-1 (phénylméthyl-4 pipéridinyl-1)-2 éthyl]-5 diméthyl-3,3 3*H*-indolone-2.

Sous atmosphère d'argon on chauffe au reflux, pendant 2h, un mélange de 5,42 g (22,8 mmoles) de chloroacétyl-5 diméthyl-3,3 3*H*-indolone-2, 3,15 g (22,8 mmoles) de carbonate de potassium sec, 4 g, soit 4 ml (22,8 mmoles) de phénylméthyl-4 pipéridine et 50 ml d'éthanol. On refroidit le mélange par un bain de glace, on ajoute 5 ml d'eau et 10 g de borohydrure de potassium et on poursuit l'agitation pendant une nuit à température ambiante. On ajoute environ 200 ml d'eau puis 300 ml d'acétate d'éthyle, on sépare la phase organique, on extrait la phase aqueuse avec deux fois 200 ml d'acétate d'éthyle, on réunit les phases organiques, on lave à l'eau, sèche sur sulfate de sodium et évapore. On obtient 8 g de cristaux bruns. Après recristallisation dans l'éthanol, lavage à l'éthanol et séchage, on isole finalement 3,57 g de cristaux blancs.
Point de fusion. 178-179 °C.

Exemple 4 (Composé n° 23).

(±)Ethyl-1 [hydroxy-1 [[(méthyl-4 phényl)méthyl]-4 pipéridinyl-1]-2 éthyl]-5 diméthyl-3,3 3*H*-indolone-2.

Sous atmosphère d'argon on chauffe au reflux pendant 1h30 un mélange de 5,2 g (20 mmoles) de chloroacétyl-5 diméthyl-3,3 éthyl-1 3*H*-indolone-2, 2,82 g (20,4 mmoles) de carbonate de potassium sec, 3,86 g (20,4 mmoles) de [(méthyl-4 phényl)méthyl]-4 pipéridine et 50 ml d'éthanol.
On laisse refroidir le mélange, on ajoute 10 ml d'eau puis 10,5 g de borohydrure de potassium, on l'agite pendant 4h à température ambiante et on ajoute 150 ml d'eau.
On filtre le précipité obtenu, on le sèche en présence de pentoxyde de phosphore, et on obtient 7,39 g de cristaux orangés qu'on purifie par chromatographie sur colonne de silice en éluant avec un mélange 97/3 de dichlorométhane/méthanol. On obtient 5,95 g d'huile qu'on reprend avec 30 ml d'éthanol chaud, on filtre la solution et on laisse refroidir le filtrat. Il se forme un précipité qu'on sépare par filtration, lave à l'éthanol et sèche à 80 °C sous vide. On isole finalement 3,37 g de cristaux blancs.
Point de fusion : 131-132 °C.

Exemple 5 (Composé n° 31).

(±)[[[Bis(fluoro-4 phényl)méthyl]-4 pipéridinyl-1]-2 hydroxy-1 éthyl]-6 dihydro-3,4 1*H*-quinoléinone-2, fumarate neutre.

On chauffe au reflux pendant 1h30 un mélange de 1,72 g (7,72 mmoles) de chloroacétyl-6 dihydro-3,4 1*H*-quinoléinone-2, 2,5 g (7,72 mmoles) de chlorhydrate de [bis(fluoro-4 phényl)méthyl]-4 pipéridine, 1,5 g de carbonate de sodium et 50 ml d'éthanol.
On refroidit le mélange par un bain de glace, on ajoute 4 ml d'eau puis 4,8 g de borohydrure de potassium, on agite à température ambiante pendant 12h, on ajoute 100 ml d'eau, on agite encore pendant 10mn et on sépare le précipité par filtration. On le lave à l'eau puis à l'hexane et on le sèche en présence de pentoxyde de phosphore. On obtient 3,36 g de base qu'on dissout dans 30 ml d'éthanol, on sépare un léger insoluble par filtration, et on ajoute 0,8 g d'acide fumarique. On chauffe le mélange au reflux pendant 15mn, on laisse refroidir et on le place dans un bain de glace. On filtre les cristaux formés (1,3 g) et on les recristallise dans 70 ml de propanol. On isole finalement 1,08 g de fumarate neutre.
Point de fusion : 161-163 °C.

Exemple 6 (Composé n° 33).

(±)Erythro [Hydroxy-1 [spiro(dihydro-2,3 benzofuranne 2:4'-pipéridinyl-1']-2 propyl]-6 dihydro-3,4 1*H*-quinoléinone-2.

On chauffe au reflux pendant 6h un mélange de 4 g (17 mmoles) de (chloro-2 propanoyl)-6 dihydro-3,4 1*H*-quinoléinone-2, 3,2 g (17 mmoles) de spiro(dihydro-2,3 benzofuranne-2:4'-pipéridine, 50 ml d'éthanol et 2 g de carbonate de sodium. On laisse refroidir, on sépare le précipité minéral par filtration, on le lave à l'éthanol, on ajoute 50 ml d'acide acétique au filtrat puis, peu à peu, 7 g de borohydrure de potassium. On agite le mélange pendant 12h, on ajoute 200 ml d'eau et de glace et 70 ml d'ammoniaque concentrée, et on extrait le mélange à l'acétate d'éthyle. On sépare, lave, sèche et évapore la phase organique, et on reprend le résidu gommeux avec 50 ml d'éthanol, on agite le mélange pendant 1h à température ambiante, on filtre le précipité blanc (2,7 g) et on le recristallise dans 75 ml d'éthanol. On isole finalement 1,9 g de composé.
Point de fusion : 195-196 °C.

Exemple 7 (Composé n° 28).

(±)[Hydroxy-1 [(naphtyl-1)oxy-4 pipéridinyl-1]-2 éthyl]-6 dihydro-3,4 1*H*-quinoléinone-2.

On chauffe au reflux pendant 2h un mélange de 3,35 g (15 mmoles) de chloroacétyl-6 dihydro-3,4 1*H*-quinoléinone-2, 4 g (15 mmoles) de chlorhydrate de (naphtyl-1)oxy-4 pipéridine, 80 ml d'éthanol et 3 g de carbonate de sodium.
On refroidit le mélange, on ajoute 10 ml d'eau et 8 g de borohydrure de potassium, on agite pendant 12h, on ajoute 150 ml d'eau, on agite le mélange pendant 30 mn, on filtre le précipité, on le sèche en présence de pentoxyde de phosphore et on purifie les 5,5 g de produit ainsi obtenus par chromatographie sur colonne de silice en éluant avec un mélange 9/1 de dichlorométhane/méthanol.
On obtient 4 g de produit qu'on recristallise dans 100 ml d'éthanol. On isole finalement 3,68 g de composé.
Point de fusion : 158-159 °C.

Exemple 8 (Composé N° 18).

(±)[Hydroxy-1 [(pyridinyl-2 oxy)-4 pipéridinyl-1]-2 éthyl]-5 3*H*-indolone-2.

a) Chloroacétyl-5 3*H*-indolone-2.

On opère comme décrit dans l'exemple 1a).

b) (Pyridinyl-2 oxy)-4 pipéridine.

On chauffe à 100 °C pendant 1h un mélange de 60,65 g (318 mmoles) de phénylméthyl-1 pipéridinol-4, 500 ml de diméthylformamide, 46,25 g, soit 41 ml (475 mmoles) de fluoro-2 pyridine et 17 g d'hydrure de sodium à 50% dans de l'huile minérale. On refroidit le mélange dans un bain de glace, on ajoute 20 ml d'eau, on l'agite pendant 30mn,et on le concentre jusqu'à un volume résiduel d'environ 200 ml. On ajoute 1 l d'eau glacée, on agite à 0 °C pendant 30mn, et on filtre, lave et sèche le précipité. On isole ainsi 90,71 g de phénylméthyl-1 (pyridinyl-2 oxy)-4 pipéridine. Point de fusion : 75 °C.

On en prélève 45 g qu'on soumet à hydrogénation dans un flacon de Parr, dans 250 ml d'éthanol et 60 ml d'acide chlorhydrique 1N, en présence de 2,5 g de charbon palladié à 10%, à 50 °C, sous une pression d'hydrogène d'environ 0,35 MPa, pendant 4h. On sépare le catalyseur par filtration, on ajoute 60 ml d'acide chlorhydrique 4N au filtrat, on évapore ce dernier, on reprend le résidu avec de l'éthanol et on l'évapore, on ajoute 100 ml de propanol-2 au résidu et, après agitation, on sépare les cristaux par filtration, on les lave avec du propanol-2 et on les sèche. On obtient 34,38 g de dichlorhydrate sous forme de cristaux blancs.
Point de fusion : 192-194 °C.

c) (±)[Hydroxy-1 [(pyridinyl-2 oxy)-4 pipéridinyl-1]-2 éthyl]-5 3*H*-indolone-2.

On chauffe au reflux pendant 2h30 un mélange de 4,19 g (20 mmoles) de chloroacétyl-5 3*H*-indolone-2, 150 ml d'éthanol, 6 g de carbonate de sodium et 5 g de dichlorhydrate de (pyridinyl-2 oxy)-4 pipéridine. On

refroidit le mélange dans un bain glacé, on ajoute 10 ml d'eau puis 8 g de borohydrure de potassium et on maintient l'agitation à température ambiante pendant 1h. On ajoute 300 ml d'eau, on extrait avec de l'acétate d'éthyle, on évapore l'extrait et on le purifie par chromatographie sur colonne de silice en éluant avec un mélange 96/4 de dichlorométhane/méthanol. Après recristallisation dans le propanol-2 on isole 1,88 g de composé pur.
Point de fusion : 164-165 °C.

Exemple 9 (Composé N° 44).

(±)[[[(Fluoro-4 phényl)méthoxy]-4 pipéridinyl-1]-2 hydroxy-1 éthyl]-6 dihydro-3,4 1*H*-quinoléinone-2.

On chauffe au reflux pendant 3h un mélange de 4,47 g (20 mmoles) de chloroacétyl-6 dihydro-3,4 1*H*-quinoléinone, 5,98 g (20 mmoles) d'oxalate de [(fluoro-4 phényl)méthoxy]-4 pipéridine, 8 g de carbonate de sodium, 180 ml d'éthanol et 20 ml d'eau. On laisse refroidir, on ajoute 10 g de borohydrure de potassium, on agite le mélange pendant 4h à température ambiante, on évapore le solvant jusqu'à un volume résiduel d'environ 80 ml, on ajoute 30 ml d'eau, on agite le mélange pendant 15mn, on sépare le précipité par filtration, on l'essore, on le sèche et on le recristallise dans 50 ml d'éthanol. On isole finalement 3,08 g de cristaux.
Point de fusion : 158-159 °C.

Exemple 10 (Composé N° 59).

(±)Erythro [[[(Fluoro-4 phényl)méthyl]-4 pipéridinyl-1]-2 hydroxy-1 propyl]-7 tétrahydro-1,3,4,5 benzo[*b*]-azépinone-2.

a) (Chloro-2 oxo-1 propyl)-7 tétrahydro-1,3,4,5 benzo[*b*]azépinone-2.

On agite pendant 30mn, à température ambiante, un mélange de 28 g (210 mmoles) de chlorure d'aluminium, 16,6 g, soit 13 ml, (130 mmoles) de chlorure de chloro-2 propanoyle et 20 ml de dichlorométhane.

On ajoute ensuite, par petites portions, 11,7 g (73 mmoles) de tétrahydro-1,3,4,5 benzo[*b*]azépinone-2 et on chauffe le mélange au reflux pendant 3h. On le laisse refroidir, on le verse sur 600 ml de glace et d'eau, on agite pendant 30mn, on sépare le solide par filtration, on le lave à l'eau et à l'hexane et on le sèche. On obtient 16,4 g de produit qu'on utilise tel quel dans l'étape suivante. Point de fusion : 136 °C.

b) (±)Erythro [[[(Fluoro-4 phényl)méthyl]-4 pipéridinyl-1]-2 hydroxy-1 propyl]-7 tétrahydro-1,3,4,5 benzo[*b*]-azépinone-2.

On chauffe au reflux pendant 8h un mélange de 3,77 g (15 mmoles) de (chloro-2 oxo-1 propyl)-7 tétrahydro-1,3,4,5 benzo[*b*]azépinone-2, 4,7 g (15 mmoles) de benzoate de [(fluoro-4 phényl)méthyl]-4 pipéridine, 3 g de carbonate de sodium et 200 ml d'éthanol. On laisse refroidir, on ajoute 50 ml d'acide acétique et 10 ml d'eau puis, peu à peu, 10 g de borohydrure de potassium. On agite le mélange pendant une nuit à température ambiante, on ajoute 250 ml d'eau glacée puis, tout en refroidissant, 70 ml d'ammoniaque concentrée.
On agite pendant 15mn, on filtre le solide, on le reprend avec du dichlorométhane et de l'eau, on sépare la phase organique, on la sèche sur sulfate de sodium, on l'évapore et on recristallise le résidu dans le propanol. On isole finalement 0,95 g de produit cristallisé.
Point de fusion : 195-196 °C.

Exemple 11 (Composé N° 76).

(±)Erythro [Hydroxy-1 [(phénylméthyl)-4 pipéridinyl-1]-2 propyl]-5 1*H*-benzimidazolone-2.

a) (Chloro-2 oxo-1 propyl)-5 1*H*-benzimidazolone-2.

On agite pendant 15mn, à température ambiante, une suspension de 80 g (600 mmoles) de chlorure d'aluminium et 50,79 g, soit 39,83 ml, (400 mmoles) de chlorure de chloro-2 propanoyle dans 120 ml de dichlorométhane.

Puis on ajoute, par petites portions, 26,82 g (200 mmoles) de 1*H*-benzimidazolone-2 et, l'addition terminée, on chauffe le mélange au reflux pendant 1h.
Après refroidissement on verse le mélange sur 1,5 l de glace et d'eau, on agite pendant 30mn, on sépare le solide par filtration, on le lave à l'eau et on le sèche. On obtient 47,2 g de cristaux gris qu'on utilise tels quels dans l'étape suivante.

b) (±)Erythro [Hydroxy-1 [(phénylméthyl)-4 pipéridinyl-1]-2 propyl]-5 1*H*-benzimidazolone-2.

On chauffe au reflux pendant 5h un mélange de 4,49 g (20 mmoles) de (chloro-2 oxo-1 propyl)-5 1*H*-benzimidazolone-2, 100 ml d'éthanol, 2 g de carbonate de sodium et 3,5 g, soit 3,52 ml (20 mmoles) de phénylméthyl-4 pipéridine.
Après refroidissement on ajoute au mélange 50 ml d'acide acétique, puis 11 g de borohydrure de potassium par petites portions. On maintient l'agitation pendant une nuit, on ajoute 200 ml d'eau, puis de l'ammoniaque concentrée jusqu'à pH basique, on extrait le mélange deux fois avec de l'acétate d'éthyle, on lave la phase organique à l'eau, on la sèche sur sulfate de magnésium et on évapore le solvant. On obtient 6,43 g de résidu qu'on purifie par chromatographie sur colonne de silice en éluant avec un mélange 98/2 de dichlorométhane/ méthanol. Après recristallisation dans l'éthanol on isole finalement 1,39 g de cristaux blancs.
Point de fusion : 221-222 °C.

Exemple 12 (Composé N°83).

(±)[Hydroxy-1 [(phénoxyméthyl)-4 pipéridinyl-1]-2 éthyl]-6 méthyl-3 dihydro-3,4 1*H*-quinazolinone-2.

On chauffe au reflux pendant 2h un mélange de 3 g (12,6 mmoles) de chloroacétyl-6 méthyl-3 dihydro-3,4 1*H*-quinazolinone-2, 2,4 g de (phénoxyméthyl)-4 pipéridine, 1,7 g de carbonate de sodium, 70 ml d'éthanol et 15 ml d'eau. On refroidit le mélange, on ajoute lentement 6 g de borohydrure de potassium, et on l'agite en le laissant revenir à température ambiante. On ajoute 125 ml d'eau, on agite pendant 1h, puis on filtre le solide, on le lave à l'eau, et on le recristallise dans l'éthanol. On isole finalement 5,1 g de composé.
Point de fusion : 205 °C.

Le tableau ci-après illustre les structures chimiques et les propriétés physiques de quelques composés selon l'invention.

Tableau

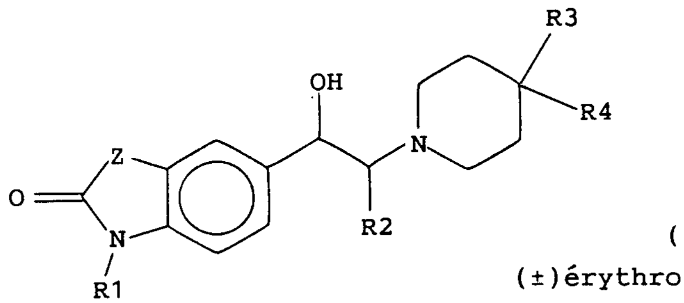

(I)

(±) si R2=H

(±)érythro si R2=$CH_3$

| N° | Z | R1 | R2 | R3 | R4 | Sel/ base | F(°C) |
|---|---|---|---|---|---|---|---|
| 1 | $-CH_2-$ | H | H | 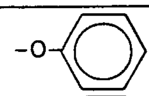 | H | base | 182-183 |
| 2 | $-CH_2-$ | H | H | 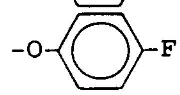 | H | base | 165-167 |
| 3 | $-CH_2-$ | H | H | 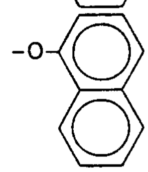 | H | base | 200-201 |
| 4 | $-CH_2-$ | H | $CH_3$ | 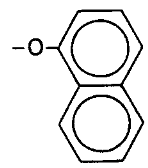 | H | base | 157-158 |
| 5 | $-CH_2-$ | H | H | 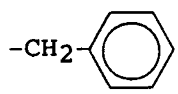 | H | base | 178-179 |
| 6 | $-CH_2-$ | H | H | 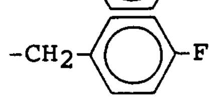 | H | base | 167-168 |
| 7 | $-CH_2-$ | H | H | 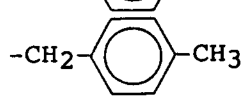 | H | base | 205-206 |
| 8 | $-CH_2-$ | H | $CH_3$ | 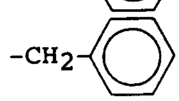 | H | base | 195-197 |
| 9 | $-CH_2-$ | H | $CH_3$ | 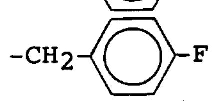 | H | base | 183-184 |
| 10 | $-CH_2-$ | H | $CH_3$ | 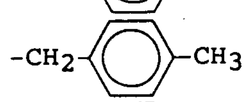 | H | base | 187-189 |
| 11 | $-CH_2-$ | H | $CH_3$ | 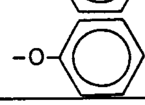 | H | base | 150-151 |

Tableau (suite)

| N° | Z | R1 | R2 | R3 | R4 | Sel/ base | F(°C) |
|---|---|---|---|---|---|---|---|
| 12 | $-CH_2-$ | H | $CH_3$ | 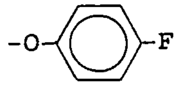 | H | base | 154-155 |
| 13 | $-CH_2-$ | H | H | 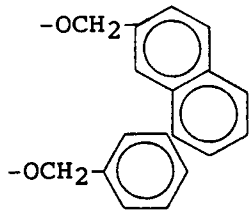 | H | base | 194-195 |
| 14 | $-CH_2-$ | H | H | | H | base | 202-203 |
| 15 | $-CH_2-$ | H | H | 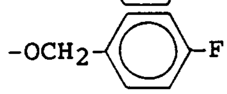 | H | base | 183-184 |
| 16 | $-CH_2-$ | H | H | 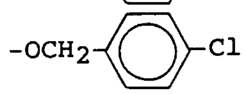 | H | base | 184-185 |
| 17 | $-CH_2-$ | H | H | 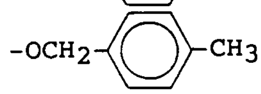 | H | base | 162-163 |
| 18 | $-CH_2-$ | H | H | 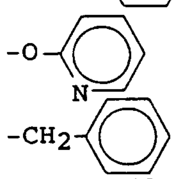 | H | base | 164-165 |
| 19 | $-C(CH_3)_2-$ | H | H | | H | base | 178-179 |
| 20 | $-C(CH_3)_2-$ | H | H | 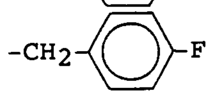 | H | base | 190-191 |
| 21 | $-C(CH_3)_2-$ | H | H | 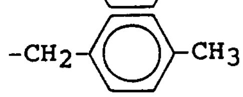 | H | base | 178-180 |
| 22 | $-C(CH_3)_2-$ | $C_2H_5$ | H | 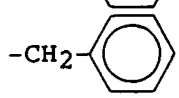 | H | base | 109-110 |
| 23 | $-C(CH_3)_2-$ | $C_2H_5$ | H | 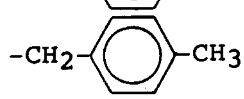 | H | base | 131-132 |
| 24 | $-CH=CH-$ | H | H | 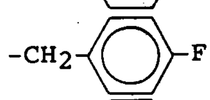 | H | base | 234-235 |
| 25 | $-(CH_2)_2-$ | H | H | 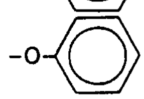 | H | base | 191-192 |
| 26 | $-(CH_2)_2-$ | H | $CH_3$ | 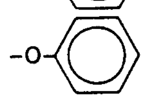 | H | base | 210-211 |
| 27 | $-(CH_2)_2-$ | H | $CH_3$ | 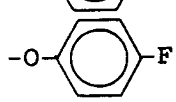 | H | base | 186-187 |

Tableau (suite)

| N° | Z | R1 | R2 | R3 | R4 | Sel/ base | F(°C) |
|---|---|---|---|---|---|---|---|
| 28 | $-(CH_2)_2-$ | H | H | 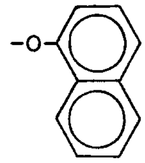 | H | base | 158-159 |
| 29 | $-(CH_2)_2-$ | H | H | 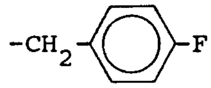 | H | base | 173-174 |
| 30 | $-(CH_2)_2-$ | H | H | 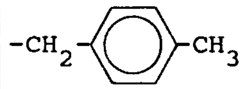 | H | base | 205-206 |
| 31 | $-(CH_2)_2-$ | H | H | 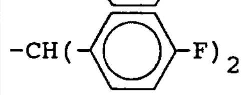 | H | fum. | 161-163 |
| 32 | $-(CH_2)_2-$ | H | H | 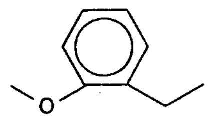 | | base | 211-212 |
| 33 | $-(CH_2)_2-$ | H | $CH_3$ | 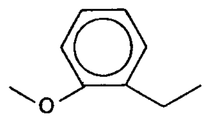 | | base | 195-196 |
| 34 | $-(CH_2)_2-$ | $C_2H_5$ | H | 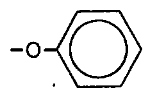 | H | base | 91-93 |
| 35 | $-(CH_2)_2-$ | $C_2H_5$ | H | 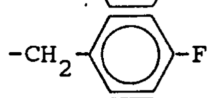 | H | base | 100-103 |
| 36 | $-(CH_2)_2-$ | $C_2H_5$ | H | 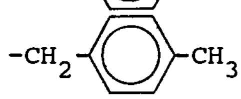 | H | base | 104-105 |
| 37 | $-(CH_2)_2-$ | H | H | 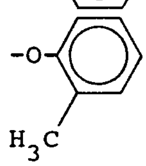 | H | base | 158-159 |
| 38 | $-(CH_2)_2-$ | H | $CH_3$ | 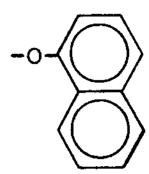 | H | base | 169-170 |

Tableau (suite)

| N° | Z | R1 | R2 | R3 | R4 | Sel/ base | F(°C) |
|---|---|---|---|---|---|---|---|
| 39 | $-(CH_2)_2-$ | H | H | 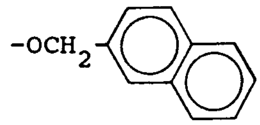 | H | base | 204-205 |
| 40 | $-(CH_2)_2-$ | H | $CH_3$ | 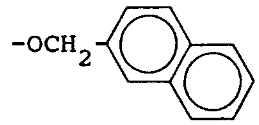 | H | base | 175-176 |
| 41 | $-(CH_2)_2-$ | H | H | 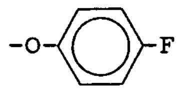 | H | base | 176-177 |
| 42 | $-(CH_2)_2-$ | H | H | 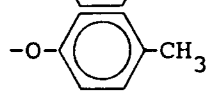 | H | base | 190-191 |
| 43 | $-(CH_2)_2-$ | H | H | 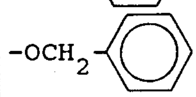 | H | base | 171-172 |
| 44 | $-(CH_2)_2-$ | H | H | 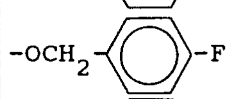 | H | base | 158-159 |
| 45 | $-(CH_2)_2-$ | H | H | 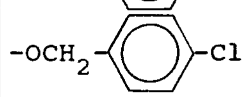 | H | base | 187-188 |
| 46 | $-(CH_2)_2-$ | H | $CH_3$ | 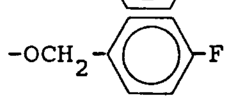 | H | base | 164-166 |
| 47 | $-(CH_2)_2-$ | H | H | 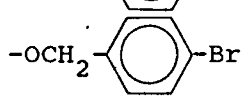 | H | base | 187-188 |
| 48 | $-(CH_2)_2-$ | H | H | 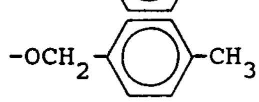 | H | base | 158-160 |
| 49 | $-(CH_2)_2-$ | H | H | 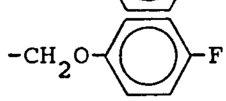 | H | base | 167-168 |
| 50 | $-(CH_2)_2-$ | H | H | 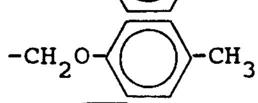 | H | base | 199-200 |
| 51 | $-(CH_2)_2-$ | H | H | 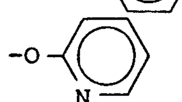 | H | base | 202-203 |
| 52 | $-(CH_2)_3-$ | H | H | 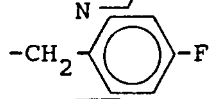 | H | base | 186-188 |
| 53 | $-(CH_2)_3-$ | H | H | 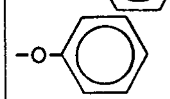 | H | base | 192-193 |
| 54 | $-(CH_2)_3-$ | H | H | 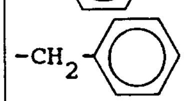 | H | base | 173-174 |

Tableau (suite)

| N° | Z | R1 | R2 | R3 | R4 | Sel/ base | F(°C) |
|---|---|---|---|---|---|---|---|
| 55 | $-(CH_2)_3-$ | H | H | 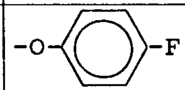 | H | base | 142-143 |
| 56 | $-(CH_2)_3-$ | H | H | 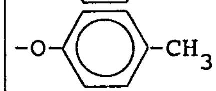 | H | base | 188-189 |
| 57 | $-(CH_2)_3-$ | H | $CH_3$ | 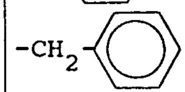 | H | base | 197-198 |
| 58 | $-(CH_2)_3-$ | H | $CH_3$ | 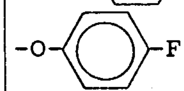 | H | base | 194-195 |
| 59 | $-(CH_2)_3-$ | H | $CH_3$ | 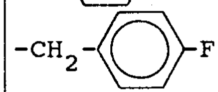 | H | base | 195-196 |
| 60 | $-(CH_2)_3-$ | H | $CH_3$ | 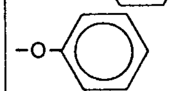 | H | base | 201-202 |
| 61 | $-(CH_2)_3-$ | H | H | 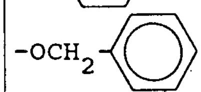 | H | base | 163-164 |
| 62 | $-(CH_2)_3-$ | H | H | 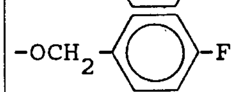 | H | base | 156-157 |
| 63 | $-(CH_2)_3-$ | H | H | 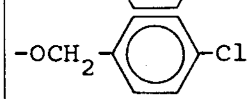 | H | base | 162-163 |
| 64 | $-(CH_2)_3-$ | H | H | 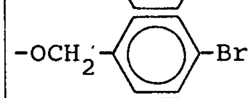 | H | base | 173-174 |
| 65 | $-(CH_2)_3-$ | H | H | 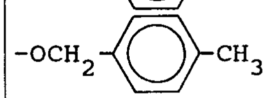 | H | base | 175-176 |
| 66 | $-(CH_2)_3-$ | H | H | 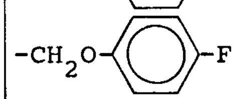 | H | base | 154-155 |
| 67 | $-(CH_2)_3-$ | H | H | 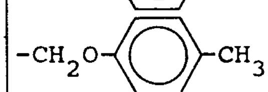 | H | base | 189-190 |
| 68 | $-(CH_2)_3-$ | H | H | 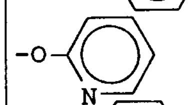 | H | base | 200 |
| 69 | -NH- | H | H | 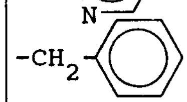 | H | base | 224-226 |
| 70 | -NH- | H | H | 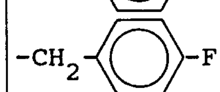 | H | base | 231-232 |
| 71 | -NH- | H | H | 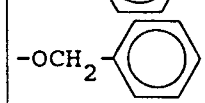 | H | base | 238-239 |

Tableau

| N° | Z | R1 | R2 | R3 | R4 | Sel/base | F(°C) |
|---|---|---|---|---|---|---|---|
| 72 | -NH- | H | H | $-OCH_2-$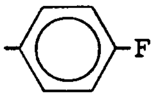-F | H | base | 237-238 |
| 73 | -NH- | H | H | $-OCH_2-$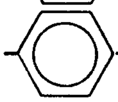-Cl | H | base | 248-250 |
| 74 | -NH- | H | H | $-OCH_2-$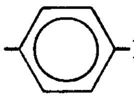-Br | H | base | 253-254 |
| 75 | -NH- | H | H | $-OCH_2-$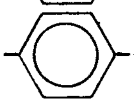$-CH_3$ | H | base | 247-248 |
| 76 | -NH- | H | $CH_3$ | $-CH_2-$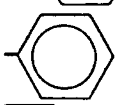 | H | base | 221-222 |
| 77 | -NH- | H | H | -O-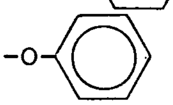 | H | base | 252-254 |
| 78 | -NH- | H | H | -O-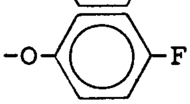-F | H | base | 240-241 |
| 79 | -NH- | H | H | -O-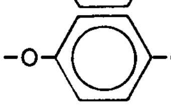$-CH_3$ | H | base | 237-238 |
| 80 | -NH- | H | H | $-CH_2O-$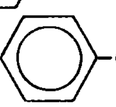$-CH_3$ | H | base | 273-275 |
| 81 | -NH- | H | H | -O-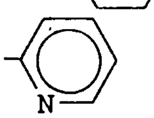 | H | base | 235-236 |
| 82 | $CH_3$<br>$-N-CH_2-$ | H | H | $-OCH_2-$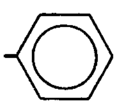 | H | base | 174 |
| 83 | $CH_3$<br>$-N-CH_2-$ | H | H | $-CH_2O-$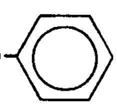 | H | base | 205 |

<u>Note</u>

Dans la colonne "Sel/base", fum. désigne le fumarate neutre.

Les composés de l'invention ont fait l'objet de diverses études pharmacologiques qui ont mis en évidence leur intérêt comme substances à activités thérapeutiques.

Ainsi, par exemple, ils ont été soumis au test de l'ischémie cérébrale globale chez la souris. L'ischémie est due à un arrêt cardiaque induit par une injection intraveineuse rapide de chlorure de magnésium. Dans ce test on mesure le "temps de survie", c'est-à-dire l'intervalle entre le moment de l'injection de chlorure

de magnésium et le dernier mouvement respiratoire observable de chaque souris. Ce dernier mouvement est considéré comme l'indice ultime d'une fonction du système nerveux central.
L'arrêt respiratoire apparaît approximativement 19 secondes après l'injection de chlorure de magnésium.
Des souris mâles (Charles River CD1) sont étudiées par groupes de 10. Elles sont nourries et abreuvées ad libitum avant les essais. Le temps de survie est mesuré 10 minutes après l'administration intrapéritonéale des composés de l'invention. Les résultats sont donnés sous la forme de la différence entre le temps de survie mesuré dans un groupe de 10 souris ayant reçu le composé et le temps de survie mesuré dans un groupe de 10 souris ayant reçu le liquide véhicule.
Les rapports entre les modifications dans le terme de survie et la dose du composé sont enregistrés graphiquement selon une courbe semilogarithmique.
Cette courbe permet le calcul de la "dose efficace 3 secondes" ($DE_{3''}$), c'est-à-dire la dose (en mg/kg) qui produit une augmentation de 3 secondes du temps de survie par rapport au groupe témoin de 10 souris non traitées.
Une augmentation de 3 secondes du temps de survie est à la fois significative statistiquement et reproductible.
Les $DE_{3''}$ des composés de l'invention sont de l'ordre de 2 à 50 mg/kg par voie intrapéritonéale.

Par ailleurs la demanderesse a trouvé qu'ils inhibent les effets stimulants du N-méthyl-D-aspartate ("NMDA") sur le taux de monophosphate-3',5' de guanosine cyclique ("cGMP") dans le cervelet du rat immature, selon un essai tel que décrit dans J. Neurochem, (1987), 49, N° 1, 195-200.
Les concentrations $CI_{50}$, qui inhibent de 50 % les effets du NMDA, sont de l'ordre de 0,3 µM pour les composés de l'invention les plus actifs dans ce test.

Enfin, un autre test effectué sur la souris a révélé que les composés de l'invention ont une activité antipsychotique intéressante.

Les essais réalisés montrent que les composés de la présente invention sont utilisables pour le traitement et la prévention de désordres cérébraux tels que ceux consécutifs à, par exemple, une attaque ischémique, un arrêt cardiaque ou respiratoire, une thrombose ou une embolie cérébrale, un traumatisme cérébral, pour le traitement de la sénilité cérébrale, de la démence consécutive aux infarctus multiples, de la démence sénile, par exemple la maladie d'Alzheimer ou la maladie de Pick, pour le traitement de l'atrophie olivo-ponto-cérébellaire et d'autres affections neurodégénératives telles que la chorée de Huntington, pour le traitement des tinnitus, pour le traitement de certains cancers, ainsi que pour le traitement d'états psychotiques tels que la schizophrénie.

A cet effet ils peuvent être présentés sous toutes formes appropriées à leur administration par voie orale ou parentérale, associés à tous excipients convenables, et dosés pour permettre une posologie journalière de 1 à 1000 mg.

## Revendications

### Revendications pour les Etats contractants suivants : AT, BE, CH, DE, FR, GB, IT, LI, LU, NL, SE

**1.** Composés, sous forme d'isomères optiques purs ou de leurs mélanges, répondant à la formule générale (I)

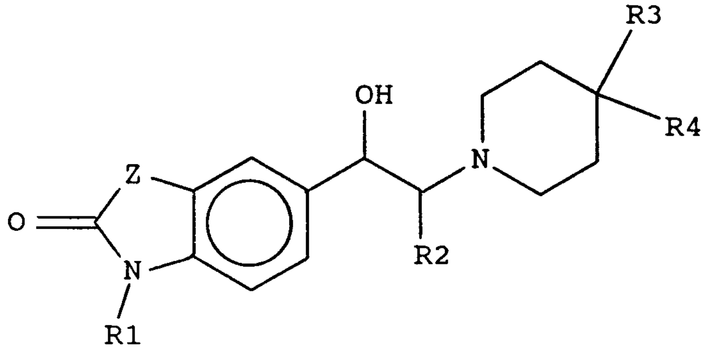

(I)

dans laquelle
Z représente un groupe de formule $-CH_2-$, $-C(CH_3)_2-$, $-CH=CH-$, $-(CH_2)_2-$, $-(CH_2)_3-$, -NH- ou $-N(CH_3)-CH_2-$ (dont l'atome d'azote est lié au groupe carbonyle),
R1 représente un atome d'hydrogène ou un groupe alkyle en $C_1$-$C_4$,

R2 représente un atome d'hydrogène ou un groupe méthyle, et
R3 représente soit un groupe phénoxy éventuellement substitué par un atome d'halogène ou un groupe méthyle, soit un groupe naphtyloxy, soit un groupe phénylméthyle substitué par un atome d'halogène ou un groupe méthyle, soit un groupe phénylméthyle non substitué lorsque Z ne représente pas un groupe de formule -CH=CH- ou $-(CH_2)_2-$, soit un groupe bis(fluoro-4 phényl)méthyle, soit un groupe phénylméthoxy éventuellement substitué par un atome d'halogène ou un groupe méthyle, soit un groupe (naphtyl-2)méthoxy, soit un groupe phénoxyméthyle éventuellement substitué par un atome d'halogène ou un groupe méthyle, soit un groupe pyridinyloxy, et
R4 représente un atome d'hydrogène, ou bien encore
R3 et R4 forment, ensemble et avec le cycle de pipéridine, un groupe spiro(dihydro-2,3 benzofuranne-2:4'-pipéridinyle-1'), ainsi que leurs sels d'addition à des acides acceptables en pharmacologie.

**2.** Procédé de préparation des composés selon la revendication 1, caractérisé en ce qu'on fait d'abord réagir une cétone halogénée de formule générale (II)

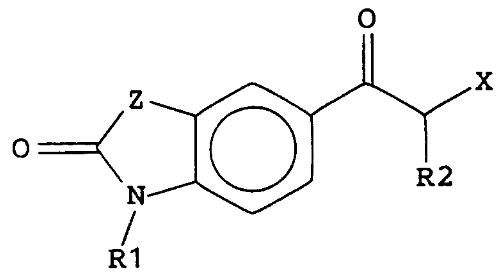

(II)

(dans laquelle Z, R1 et R2 sont tels que définis dans la revendication 1 et X représente un atome d'halogène tel que le chlore ou le brome) avec une pipéridine de formule générale (III)

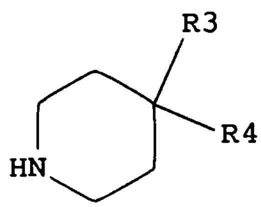

(III)

(dans laquelle R3 et R4 sont tels que définis dans la revendication 1), en présence d'une base minérale telle que le carbonate de sodium ou de potassium, ou en présence d'un excès de pipéridine de formule générale (III), dans un solvant tel qu'un alcool inférieur ou l'acétonitrile, et éventuellement en présence d'eau,
puis on réduit la cétone de formule générale (IV) ainsi obtenue

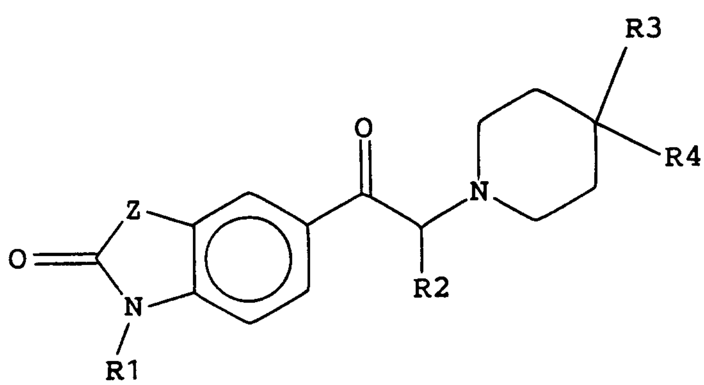

(IV)

au moyen de borohydrure de sodium ou de potassium, en milieu alcalin ou acide.

**3.** Composition pharmaceutique, caractérisée en ce qu'elle contient un composé selon la revendication 1.

**Revendication pour les Etats contractants suivants : ES, GR**

**1.** Procédé de préparation de composés répondant à la formule générale (I)

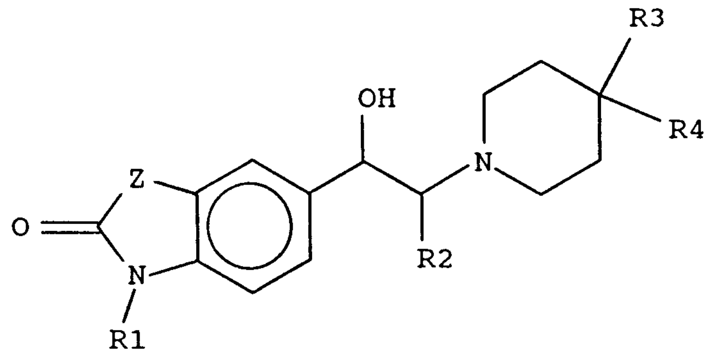

(I)

dans laquelle
Z représente un groupe de formule $-CH_2-$, $-C(CH_3)_2-$, $-CH=CH-$, $-(CH_2)_2-$, $-(CH_2)_3-$, -NH- ou $-N(CH_3)-CH_2-$ (dont l'atome d'azote est lié au groupe carbonyle),
R1 représente un atome d'hydrogène ou un groupe alkyle en $C_1$-$C_4$,
R2 représente un atome d'hydrogène ou un groupe méthyle, et
R3 représente soit un groupe phénoxy éventuellement substitué par un atome d'halogène ou un groupe méthyle, soit un groupe naphtyloxy, soit un groupe phénylméthyle substitué par un atome d'halogène ou un groupe méthyle, soit un groupe phénylméthyle non substitué lorsque Z ne représente pas un groupe de formule -CH=CH) ou $-(CH_2)_2-$, soit un groupe bis(fluoro-4 phényl)méthyle, soit un groupe phénylméthoxy éventuellement substitué par un atome d'halogène ou un groupe méthyle, soit un groupe (naphtyl-2)méthoxy, soit un groupe phénoxyméthyle éventuellement substitué par un atome d'halogène ou un groupe méthyle, soit un groupe pyridinyloxy, et
R4 représente un atome d'hydrogène, ou bien encore
R3 et R4 forment, ensemble et avec le cycle de pipéridine, un groupe spiro(dihydro-2,3 benzofuranne-2:4'-pipéridinyle-1'), procédé caractérisé en ce qu'on fait d'abord réagir une cétone halogénée de formule générale (II)

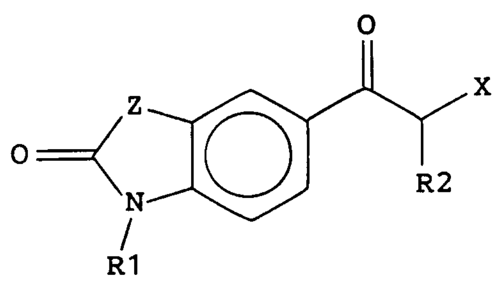

(II)

(dans laquelle Z, R1 et R2 sont tels que définis ci-dessus et X représente un atome d'halogène tel que le chlore ou le brome) avec une pipéridine de formule générale (III)

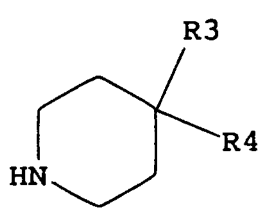

(III)

(dans laquelle R3 et R4 sont tels que définis ci-dessus) en présence d'une base minérale telle que le carbonate de sodium ou de potassium, ou en présence d'un excès de pipéridine de formule générale (III), dans un solvant tel qu'un alcool inférieur ou l'acétonitrile, et éventuellement en présence d'eau, puis on réduit la cétone de formule générale (IV) ainsi obtenue

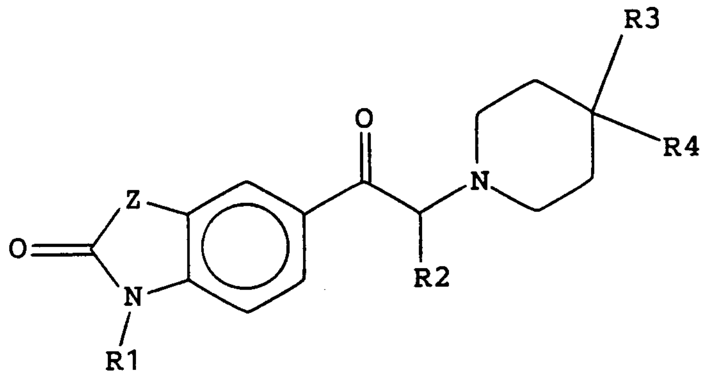

(IV)

au moyen de borohydrure de sodium ou de potassium, en milieu alcalin ou acide.

**Claims**

**Claims for the following Contracting States : AT, BE, CH, DE, FR, GB, IT, LI, LU, NL, SE**

1. Compounds, in the form of pure optical isomers or their mixtures, corresponding to the general formula (I)

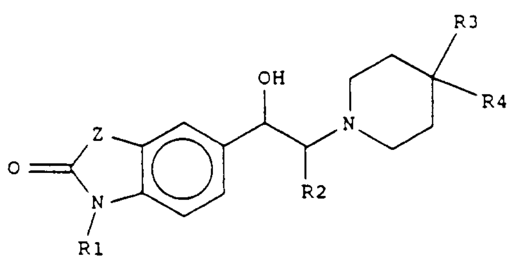

(I)

in which
Z represents a group of formula $-CH_2-$, $-C(CH_3)_2-$, $-CH=CH-$, $-(CH_2)_2-$, $-(CH_2)_3-$, -NH- or $-N(CH_3)CH_2-$ (in which the nitrogen atom is bonded to the carbonyl group),
R1 represents a hydrogen atom or a $C_1$-$C_4$ alkyl group,
R2 represents a hydrogen atom or a methyl group, and
R3 represents either a phenoxy group which may be substituted by a halogen atom or a methyl group, or a naphthyloxy group, or a phenylmethyl group substituted by a halogen atom or a methyl group, or an unsubstituted phenylmethyl group when Z does not represent a group of formula $-CH=CH-$ or $-(CH_2)_2-$, or a bis(4-fluorophenyl)methyl group, or a phenylmethoxy group which may be substituted by a halogen atom or a methyl group, or a (2-naphthyl)methoxy group, or a phenoxymethyl group which may be substituted by a halogen atom or a methyl group, or a pyridinyloxy group, and
R4 represents a hydrogen atom, or, moreover,
R3 and R4 form, together and with the piperidine ring, a spiro(2,3-dihydrobenzofuran-2,4'-piperid-1'-yl) group, as well as their addition salts with pharmacologically acceptable acids.

2. Process for the preparation of compounds according claim 1, characterized in that a halogenated ketone of general formula (II)

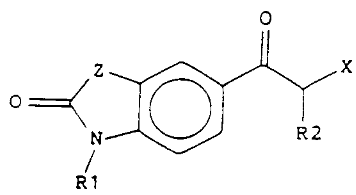

(II)

(in which Z, R1 and R2 are as defined in claim 1 and X represents a halogen atom such as chlorine or bromine) is first of all reacted with a piperidine of general formula (III)

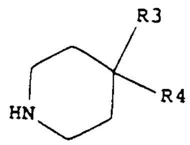

(III)

(in which R3 and R4 are as defined in claim 1), in the presence of an inorganic base such as sodium carbonate or potassium carbonate, or in the presence of an excess of the piperidine of general formula (III), in a solvent such as a lower alcohol or acetonitrile, and if necessary in the presence of water,
the ketone of general formula (IV)

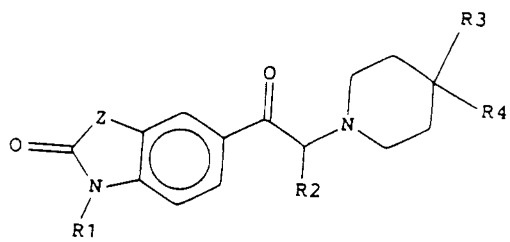

(IV)

thus obtained is reduced by means of sodium borohydride or potassium borohydride, in an alkaline or acid medium.

3. Pharmaceutical composition, characterized in that it contains a compound according to claim 1.

**Claim for the following Contracting States : ES, GR**

1. Process for the preparation of compounds corresponding to the general formula (I)

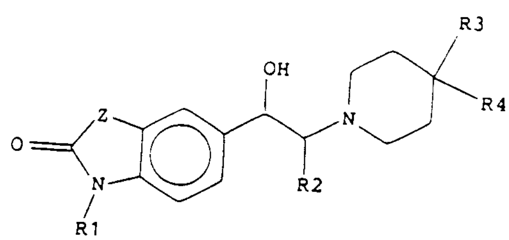

(I)

in which
Z represents a group of formula $-CH_2-$, $-C(CH_3)_2-$, $-CH=CH-$, $-(CH_2)_2-$, $-(CH_2)_3-$, -NH- or $-N(CH_3)CH_2-$ (in which the nitrogen atom is bonded to the carbonyl group),
R1 represents a hydrogen atom or a $C_1$-$C_4$ alkyl group,
R2 represents a hydrogen atom or a methyl group, and
R3 represents either a phenoxy group which may be substituted by a halogen atom or a methyl group, or a naphthyloxy group, or a phenylmethyl group substituted by a halogen atom or a methyl group, or

an unsubstituted phenylmethyl group when Z does not represent a group of formula -CH=CH- or $-(CH_2)_2-$, or a bis(4-fluorophenyl)methyl group, or a phenylmethoxy group which may be substituted by a halogen atom or a methyl group, or a (2-naphthyl)methoxy group, or a phenoxymethyl group which may be substituted by a halogen atom or a methyl group, or a pyridinyloxy group, and
R4 represents a hydrogen atom, or, moreover,
R3 and R4 form, together and with the piperidine ring, a spiro(2,3-dihydrobenzofuran-2,4'-piperid-1'-yl) group, which process is characterized in that a halogenated ketone of general formula (II)

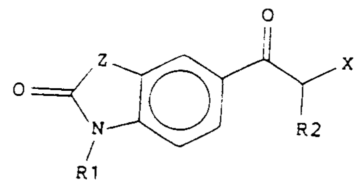

(II)

(in which Z, R1 and R2 are as defined above and X represents a halogen atom such as chlorine or bromine) is first of all reacted with a piperidine of general formula (III)

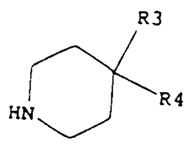

(III)

(in which R3 and R4 are as defined above), in the presence of an inorganic base such as sodium carbonate or potassium carbonate, or in the presence of an excess of the piperidine of general formula (III), in a solvent such as a lower alcohol or acetonitrile, and if necessary in the presence of water,
the ketone of general formula (IV)

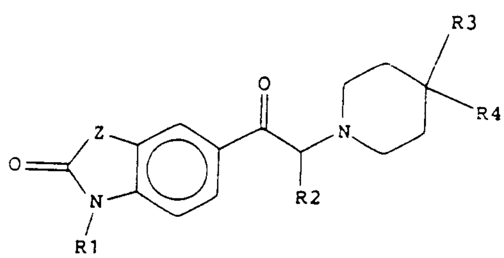

(IV)

thus obtained is reduced by means of sodium borohydride or potassium borohydride, in an alkaline or acid medium.

## Patentansprüche
**Patentansprüche für folgende Vertragsstaaten : AT, BE, CH, DE, FR, GB, IT, LI, LU, NL, SE**

1. Verbindungen in Form der reinen optischen Isomeren oder Mischungen davon, entsprechend der allgemeinen Formel (I)

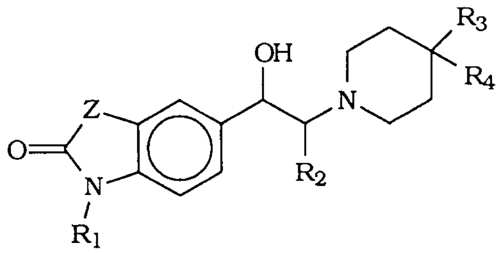

(I)

in der

Z eine Gruppe der Formel $-CH_2-$, $-C(CH_3)_2-$, $-CH=CH-$, $-(CH_2)_2-$, $-(CH_2)_3-$,-NH- oder $-N(CH_3)CH_2-$ (worin das Stickstoffatom an die Carbonylgruppe gebunden ist),

$R_1$ ein Wasserstoffatom oder eine $C_1$-$C_4$-Alkylgruppe,

$R_2$ ein Wasserstoffatom oder eine Methylgruppe und

$R_3$ entweder eine Phenoxygruppe, die gegebenenfalls durch ein Halogenatom oder eine Methylgruppe substituiert ist, oder eine Naphthyloxygruppe oder eine Phenylmethylgruppe, die durch ein Halogenatom oder eine Methylgruppe substituiert ist, oder eine nichtsubstituierte Phenylmethylgruppe, wenn Z keine Gruppe der Formel$-CH=CH-$ oder$-(CH_2)_2-$ darstellt, oder eine Bis(4-fluor-phenyl)-methylgruppe oder eine Phenylmethoxygruppe, die gegebenenfalls durch ein Halogenatom oder eine Methylgruppe substituiert ist, oder eine (2-Naphthyl)-methoxygruppe, oder eine Phenoxymethylgruppe, die gegebenenfalls durch ein Halogenatom oder eine Methylgruppe substituiert ist, oder eine Pyridinyloxygruppe, und

$R_4$ ein Wasserstoffatom bedeuten, oder

$R_3$ und $R_4$ gemeinsam mit dem Piperidinring eine Spiro-(4'-piperidin-1'-yl-2,3-dihydro-2-benzofuran)-gruppe bedeuten, sowie deren Additionssalze mit pharmakologisch annehmbaren Säuren.

2. Verfahren zur Herstellung der Verbindungen nach Anspruch 1, **dadurch gekennzeichnet,** daß man zunächst ein halogeniertes Keton der allgemeinen Formel (II)

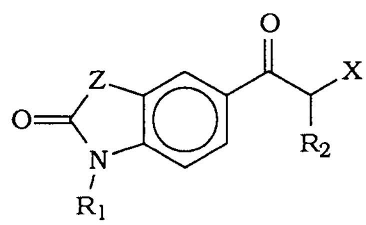

(II)

(in der Z, $R_1$ und $R_2$ die in Anspruch 1 angegebenen Bedeutungen besitzen und X ein Halogenatom, wie ein Chloratom oder ein Bromatom bedeuten) mit einem Piperidin der allgemeinen Formel (III)

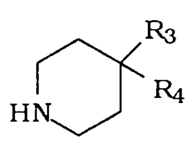

(III)

(in der $R_3$ und $R_4$ die in Anspruch 1 angegebenen Bedeutungen besitzen) in Gegenwart einer anorganischen Base, wie Natriumcarbonat oder Kaliumcarbonat, oder in Gegenwart eines Überschusses des Piperidins der allgemeinen Formel (III) in einem Lösungsmittel, wie einem niedrigmolekularen Alkohol oder Acetonitril, und gegebenenfalls in Gegenwart von Wasser umsetzt,

und dann das in dieser Weise erhaltene Keton der allgemeinen Formel (IV)

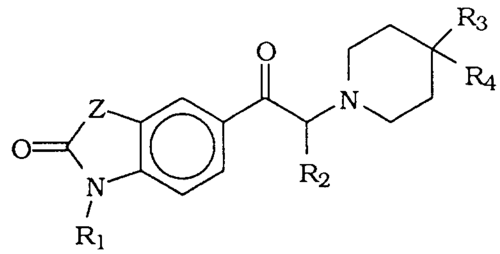

(IV)

mit Natriumborhydrid oder Kaliumborhydrid in alkalischem oder saurem Medium reduziert.

3. Pharmazeutische Zubereitung, **dadurch gekennzeichnet,** daß sie eine Verbindung nach Anspruch 1 enthält.

**Patentanspruch für folgende Vertragsstaaten : ES, GR**

1. Verfahren zur Herstellung von Verbindungen der allgemeinen Formel (I)

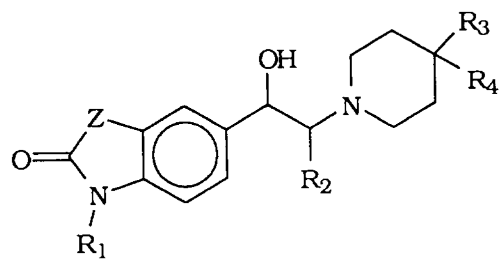

(I)

in der
Z eine Gruppe der Formel $-CH_2-$, $-C(CH_3)_2-$, $-CH=CH-$, $-(CH_2)_2-$, $-(CH_2)_3-$,-NH- oder $-N(CH_3)CH_2-$ (worin das Stickstoffatom an die Carbonylgruppe gebunden ist),
$R_1$ ein Wasserstoffatom oder eine $C_1$-$C_4$-Alkylgruppe,
$R_2$ ein Wasserstoffatom oder eine Methylgruppe und
$R_3$ entweder eine Phenoxygruppe, die gegebenenfalls durch ein Halogenatom oder eine Methylgruppe substituiert ist, oder eine Naphthyloxygruppe oder eine Phenylmethylgruppe, die durch ein Halogenatom oder eine Methylgruppe substituiert ist, oder eine nichtsubstituierte Phenylmethylgruppe, wenn Z keine Gruppe der Formel-$CH=CH$- oder-$(CH_2)_2$-darstellt, oder eine Bis(4-fluor-phenyl)-methylgruppe oder eine Phenylmethoxygruppe, die gegebenenfalls durch ein Halogenatom oder eine Methylgruppe substituiert ist, oder eine (2-Naphthyl)-methoxygruppe, oder eine Phenoxymethylgruppe, die gegebenenfalls durch ein Halogenatom oder eine Methylgruppe substituiert ist, oder eine Pyridinyloxygruppe, und
$R_4$ ein Wasserstoffatom bedeuten, oder
$R_3$ und $R_4$ gemeinsam mit dem Piperidinring eine Spiro-(4'-piperidin-1'-yl-2,3-dihydro-2-benzofuran)-gruppe bedeuten,
**dadurch gekennzeichnet,** daß man zunächst ein halogeniertes Keton der allgemeinen Formel (II)

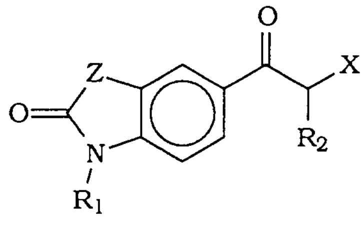

(II)

(in der Z, $R_1$ und $R_2$ die in Anspruch 1 angegebenen Bedeutungen besitzen und X ein Halogenatom, wie ein Chloratom oder ein Bromatom bedeuten) mit einem Piperidin der allgemeinen Formel (III)

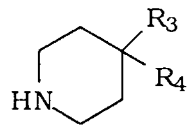

(III)

(in der $R_3$ und $R_4$ die in Anspruch 1 angegebenen Bedeutungen besitzen) in Gegenwart einer anorganischen Base, wie Natriumcarbonat oder Kaliumcarbonat, oder in Gegenwart eines Überschusses des Piperidins der allgemeinen Formel (III) in einem Lösungsmittel, wie einem niedrigmolekularen Alkohol oder Acetonitril, und gegebenenfalls in Gegenwart von Wasser umsetzt,
und dann das in dieser Weise erhaltene Keton der allgemeinen Formel (IV)

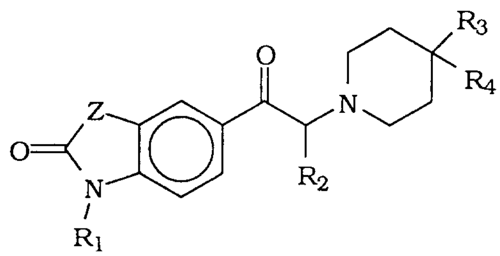

(IV)

mit Natriumborhydrid oder Kaliumborhydrid in alkalischem oder saurem Medium reduziert.